(19) Europäisches Patentamt
European Patent Office
Office européen des brevets

(11) Publication number: **0 052 645**
**B1**

(12) # EUROPEAN PATENT SPECIFICATION

(45) Date of publication of patent specification: **13.05.87**

(51) Int. Cl.$^4$: **A 61 K 49/00,** C 07 D 453/02, A 61 K 43/00, G 01 N 33/534

(21) Application number: **81901662.7**

(22) Date of filing: **27.05.81**

(86) International application number: **PCT/US81/00703**

(87) International publication number: **WO 81/03422 10.12.81 Gazette 81/29**

(54) **GAMMA-EMITTING RECEPTOR-BINDING QUINUCLIDINYL BENZILATES METHODS OF PREPARATION THEREOF AND IMAGING AND ASSAY METHODS UTILIZING SAME.**

(30) Priority: **03.06.80 US 156106**

(43) Date of publication of application: **02.06.82 Bulletin 82/22**

(45) Publication of the grant of the patent: **13.05.87 Bulletin 87/20**

(84) Designated Contracting States: **CH DE FR GB LI**

(56) References cited:
**DE-A-1 800 823**
**DE-A-2 640 209**
**GB-A- 725 228**
**US-A-3 714 357**
**US-A-3 833 592**

**CHEMICAL ABSTRACTS, vol. 91, no. 17, October 22nd, 1979, page 73, abstract 134240r Columbus, Ohio, US S.D. FLANAGAN et al.: "An Iodine-125-labeled binding probe for the muscarinic cholinergic receptor"**

**The file contains technical information submitted after the application was filed and not included in this specification**

(73) Proprietor: **Research Corporation**
**405 Lexington Avenue**
**New York, N.Y. 10017 (US)**

(72) Inventor: **ECKELMAN, William C.**
**2 Harvard Court**
**Rockville, MD 20850 (US)**
Inventor: **REBA, Richard C.**
**1121 University Blvd. West**
**Silver Spring, MD 20902 (US)**
Inventor: **RZESZOTARSKI, Warclaus J.**
**4607 Brandywine Street, N.W.**
**Washington, DC 20016 (US)**
Inventor: **GIBSON, Raymond E.**
**2601 North Franklin Road**
**Arlington, VA 22201 (US)**

(74) Representative: **Hansmann, Axel et al**
**Patentanwälte HANSMANN & VOGESER**
**Albert-Rosshaupter-Strasse 65**
**D-8000 München 70 (DE)**

**EP 0 052 645 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 92, no. 22, June 6th, 1980, page 322, abstract 18274b Columbus, Ohio, US W.C. ECKELMAN et al.: "The design of receptor binding radiopharmaceuticals"

CHEMICAL ABSTRACTS, vol. 91, no. 1, January 1st, 1980, page 251, abstract 34055j, Columbus, Ohio, US, W.C. ECKELMAN et al.: "Receptor-binding radiotracers: a class of potential radiopharmaceuticals"

CHEMICAL ABSTRACTS, vol. 91, no. 23, December 12th, 1979, page 256, abstract 188752c, Columbus, Ohio, US, W.C. ECKELMAN et al.: "The distribution of the muscarinic acetylcholine receptor antagonists, quinuclidinyl benzilate and quinuclidinyl benzilate methiodide (both tritiated), in rat, guines pig, and rabbit"

**Description**

BACKGROUND OF THE INVENTION

Field of the Invention

The present invention relates to various muscarinic cholinergic receptor-binding compounds of formula I, gamma-emitting radio-isotope derivatives thereof, intermediates and methods for the production thereof and the use of the said radioisotope derivatives as radio-pharmaceuticals in the assay and external imaging of myocardial tissue and other organs containing muscarinic cholinergic receptors.

It is known that muscarinic cholinergic receptors are concentrated in myocardial and other tissue. It is theorized that these receptors are involved in the action of drugs or biochemicals in the myocardial tissue and that changes in the concentration of the receptor in the myocardial tissue are a function of a disease state therein or in other tissue containing the receptors.

It has been established that various compounds function both in vitro and in vivo as muscarinic cholinergic receptor binders or antagonists. These compounds have a high affinity for and competitively bind with the receptor. It has been established that various quinuclidinyl benzilates are effective muscarinic cholinergic receptor binders. It has further been suggested that tritium-labeled quinuclidinyl benzilates may be utilized as radiotracers for various assay procedures involving myocardial tissue. See Eckelman et al, J. Nucl. Med. *20,* 350 (1979) and Gibson et al, J. Nucl. Med. *20,* 865 (1979). CA 91, N. 17, (1979) page 17 No. 1342402 describes $^{125}$I-labeled quinuclidinyl compounds used as binding probes for muscarinic cholinergic receptors. These compounds, however, show a high level of non-specific binding, and very little of the desired receptor binding.

There are numerous disadvantages inherent in the use of tritium-labeled radiotracers. For example, numerous problems are associated with "counting" beta-emissions of tritium-labeled compounds. Liquid scintillator must be added to each sample which is a time-consuming and expensive procedure. Toluene is the typical scintillator liquid employed which is presently subject to strict, environmental sanctions thereby rendering its disposal problematical. In addition beta-counting procedures are plagued with problems of chemluminescense and quenching which are absent in gamma-counting.

T1-201 is presently employed for the detection and quantification of myocardial infarcts. However, the radiohalogenides such as I-123 and Br-77 have better imaging characteristics in that their higher gamma energies can be detected with increased sensitivity and positional resolution as compared with the lower gamma energy of T1-201 (80 Ke V X-rays).

Since the size of the infarct is related to mortality and residual function, improved resolution will mean improved prognosis and evaluation of drug therapy. The radiohalogenides F-18 and Br-75 are positron emitters which offer the unique capabilities of reduction and quantification associated with such decay characteristics.

It is an object of the present invention to provide various quinuclidinyl benzilates and gamma-emitting radioisotope containing quinuclidinyl benzilates useful for muscarinic cholinergic receptor assays, and imaging of the myocardium and other muscarinic cholinergic assay and tissue imaging techniques.

Summary of the Invention

These and other objects of the present invention are achieved by providing compounds of the formula I:

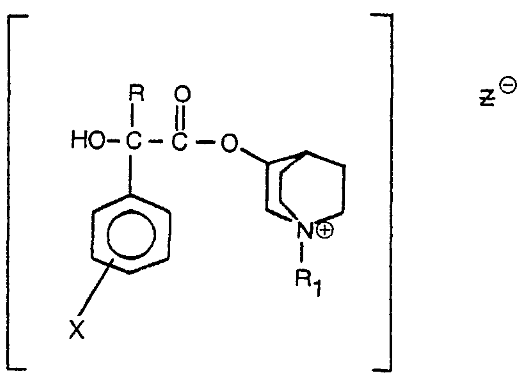

Wherein

R is phenyl, cyclopentyl or cyclohexyl,

$R_1$ is H or $CH_3$,

X is in the ortho-, meta- or para- position, and is selected from $^{125}I$, $^{123}I$, I, $^{18}F$, $^{77}Br$, $^{75}Br$, $NH_2$, and

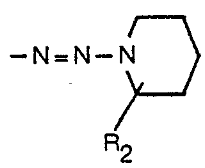

wherein $R_2$ is in the 2, 3, or 4, position and is selected from H and $CH_3$, and,

~~Z~~$^{\ominus}$ is an anion; or the corresponding free amines, i.e., the non-protonated or non-quaternary salt form.

The radio-labeled compounds are gamma-emitting and are useful in formulating compositions suitable for the *in vitro* assay muscarinic cholinergic receptors in e.g., myocardial tissue and other tissue containing muscarinic cholinergic receptors.

The iodo, amino- and triazeno-derivatives are valuable as intermediates in the preparation of the radio-labeled analogs.

Detailed Description of the Invention

The aminobenzil may be prepared according to the method of Augl et al, *Annu, Conf.*, SPE, Reinf. Plastic/Compos. Div. Proc., 26th, 19D, 1 (1971) to produce 4-Aminobenzil. The 4-Aminobenzil is rearranged to yield 4-aminobenzilic acid which is in turn esterfied to produce the ethyl ester. The ethyl ester of the 4-aminobenzilic acid is then reacted with quinuclidin-3-ol to give the expected 3-quinuclidinyl 4-aminobenzilate (4-amino-QNB). The purified 4-amino QNB is then converted to the 4-triazeno-QNB according to the procedure reported by Tewson et al, J. Nucl. Med. *20*, 671 (1979). The purified 4-triazeno-QNB is then reacted with iodide or a desired gamma-emitting radionuclide to produce the desired product.

In the above structural formula the anion $Z^{\ominus}$ may be any pharmaceutically acceptable anion such as $Cl^-$, $Br^-$, or $I^-$, $SO_4^=$, $HCOO^-$, $CH_3COO^-$.

The following examples illustrate the preparation of the claimed compounds.

Example 1

4-Aminobenzilic Acid

A solution of 25 g of NaOH in 50 ml of $H_2O$ was placed in a water bath kept at 95°C. To this magnetically stirred solution was added 11.26 g (50 mol) of 4-amino-benzil (I) in small portions. After the addition was completed the mixture was stirred for 5 hours at 95°C. Water was added during the reaction to maintain the volume at about 100 ml. After 5 hours the heating was discontinued and the reaction mixture was transferred to a separatory funnel and extracted twice with 50 ml of ethyl ether. The aqueous layer was cooled to 0°C acidified with conc. $H_2SO_4$ until turbid, and extracted with ethyl acetate (AcOEt) (100 ml). The aqueous layer was acidified and extracted with AcOEt again. The combined AcOEt layers were washed with water (2x), dried over $MgSO_4$, filtered and spin evaporated in AcOEt and filtered. The combined precipitates were recrystallized from water/acetone 25/75. Yield 8.9 g (73%). Yellow crystals, mp dec. 150°C; Silica gel TLC in acetone, Rf 0.25; HPLC Bondapak $\mu C_{18}$ in $MeOH/H_2O$ (75.25) pH 4 (formic acid). IR, UV, mass spec. were consistent with the structure. Elemental Analysis calc for $C_{14}H_{13}NO_3 \cdot 2/3\ H_2O$, Calc. C 67.28, H 5.78, N 5.60; Found C 67.45, 67.64, H 5.49, 5.41, N 5.52, 5.51.

Example 2

4-Aminobenzilic Acid Ethyl Ester

4.8 g (19.7 mmol) of 4-aminobenzilic acid was dissolved in 200 ml of absolute ethanol (EtOH) saturated with dry HCl gas. The reaction mixture was refluxed for 24 hours then spin evaporated to dryness in vacuum. The residue was dissolved in water and neutralized with $NaHCO_3$, extracted with 2 x 50 ml of AcOEt, washed with water (2x) and the AcOEt extract filtered through a siliconized filter and dried over $MgSO_4$. The dried solution was filtered and spin evaporated in vacuum. Yellow oil, 2.8 g (52%). SGTLC in acetone Rf 0.8. HPLC Bondapak $\mu C_{18}$ MeOH $1/H_2O$ (75.25) pH 4 (formic acid). Elemental Analysis Calc. for $C_{16}H_{17}NO_3$. Calc N 5.16; Found N 5.04, 5.26.

Example 3

4-Aminobenzilate of 3-(R,S)-quinuclidinol

5.16 g (40 mmol) of 3-(R,S)-quinuclidinol was dissolved in 50 ml of dry benzene and 20 ml of benzene distilled off. A clean 100 mg piece of sodium was added and the magnetically stirred suspension, protected from moisture and $CO_2$ (NaOH trap), refluxed for 24 hours. 2.8 g (10.3 mmol) of ethyl 4-aminobenzilate was dissolved in 50 ml of dry benzene and 20 ml of benzene removed by distillation.

Both solutions were combined and refluxed, protected from moisture and CO for 24 hours. The solution was spin evaporated, the residue suspended in water and extracted twice with 50 ml of AcOEt. The AcOEt extract was washed repeatedly with water, filtered through a siliconized filter paper and dried over $MgSO_4$. The dried solution was dissolved in $CH_3CN$ and charged on a silica gel column (2.8 x 100 cm) eluted with $CH_3CN$. Pure fractions crystallized on standing. The product recrystalized from $CH_3CN$, 1.5 g (41%) white crystals; SGTLC n-butanol, acetic acid, water 4:1:1, Rf 0.4; HPLC Bondapak $\mu C_{18}$ $MeOH/H_2O$ 40/60, pH 4 formic acid. IR, UV, mass spec. were consistent with the structure of the product. Calc. for $C_{21}H_{24}N_2O_3$. Calc. C 71.57, H 6.86, N 7.95; Found C 71.27, H 6.87, N 8.19.

Example 4

4-[2-(3-methylpiperid-1-yl)-1,2-diaza-ethylen-1-yl]-benzilate of 3-(R,S)-quinuclidinol

Sodium nitrite (75 mg, 1.08 mmol) was added to a cooled (0°C) solution of 190 mg (54 mmol) of 4-amino QNB (IV) in 6 ml of 10% $H_2SO_4$ and acetone (5:1). The mixture was stirred for 15 min. at 0° then treated with 65 mg (1.08 mmol) of urea.

The diazonium salt slurry was added to a cooled (0°C) solution of 536 mg (5.4 mmol) of 3-methylpiperidine in 5 ml of water. The mixture was stirred at 0°C for 20 min. then made basic with 4N NaOH to pH 12 and extracted with $CHCl_3$ (3x5 ml). The combined extracts were washed repeatedly with water and dried over $MgSO_4$. The solution was filtered and spin evaporated in vacuum. The residue was extracted with petroleum ether (3 x 10 ml) and the combined extracts evaporated and dried in high vacuum to remove the traces of 3-methylpiperidine. Obtained was 24 mg (96%) of viscous yellow oil. SGTLC in 2% $NH_4OH$ in MeOh, Rf 0.5 HPLC Bondapak $\mu_{18}$ 5mM 1-hexanesulfonic acid pH 4 ($H_2SO_4$) in Me OH/$H_2O$, 60/40. UV, IR, mass spec. were consistent with the product structure. Elemental analysis for $C_{27}$ $H_{34}N_4O_3 \cdot 2H_2O$. Calc. 65.03, H 7.68, N 11.23; Found C 65.35, H 7.44, N 10.62.

Example 5

(R,S)-Quinuclidin-3-ol-4-iodo benzilate

A solution of 5 mg (11 µmol) of QNB-triazene (V) and 1.6 g (11 µmol) of sodium iodide in 1 ml of trifluoroethanol was treated with 6.4 mg of methane-sulfonic acid. The reaction mixture was heated on a water bath for 45 min., cooled, 5 ml of water added and extracted with 2 x 5 ml of AcOEt. The aqueous layer was then neutralized with 4N NaOH and extracted with 3 x 5 ml of AcOEt. The organic layer was washed three times with 1 ml of water, filtered through a siliconized paper and dried over $Na_2SO_4$. After spin evaporation in vacuum, a yield of 2.7 mg (54%) was obtained as a yelow oil. UV, IR, mass spec. were consistent SGTLC in n-BuOH:AcOH:$H_2O$, 60/40. Bondapak $\mu_{18}$ 5mM 1-octanesulfonic acid, pH 4, MeOH/$H_2O$, 60/40.

Example 6

(R,S)-Quinuclidin-3-ol-4-bromo or 4-iodo or 4-fluorobenzilate (radio nuclides)

The compounds are prepared using the above procedure to produce the iodo-benzilate substituting sodium radio iodide or radio bromide or tetrabutyl ammonium radio fluoride for sodium iodide.

To prepare the protonated or quaternary derivative, the product of Example 5 or 6 is dissolved in a solution of the appropriate salt and recrystallized therefrom.

Example 7

The radiohalogenide containing compound is utilized for imaging as follows:

The radio labeled tracer (I-123-QNB) is injected intravenously in the amount of 1 to 10 mCi at a specific activity to exceed 1000 Ci/mmol. Images of the distribution of radioactivity in the target organ are obtained as a function of time and the data stored in a computer. The data is then analyzed using an appropriate pharmacokinetic model to determine the concentration of receptors as a function of time. Alternately the information can be used to determine the blood flow to the target organ. For positron-emitting radiotracers such as Br-75 and F-18, coincidence counting is used. The images are collected over 2 hours and the target organ can be any locus of muscarinic cholinergic receptors such as the brain, the heart or the pancreas.

Example 8

The radiohalogenide containing compound can be used for the radio assay of tissue as follows:

Heart microsomal preparations are obtained as described by Harden et al [Mol Pharmacol 12: 1-15, 1976]. The heart from a rabbit is removed after the animal has been killed with ether. The heart is dissected free from atria and large vessels, minced with scissors and homogenized in buffer using a Brinkman Polytron. The homogenate is certrifuged at 10,000 g for 20 min. and the supernatent discarded. The pellet is resuspended in buffer and recentrifuged. The pellet is then suspended in buffer. This homogenate is centrifuged over a layer of 0.2 M. sucrose above a layer of 1.72 M sucrose. Membranes are collected at the interface of the two sucrose layers and used immediately for the radio-receptor assay.

Aliquots of the muscarinic cholinergic receptor isolated above, I-125 IQNB and the test drugs are incubated at 37°C with agitation. Incubations are carried out routinely for 60 min. Each incubation is terminated within 10 sec. by filtering the suspension through a GF/B glass fiber filter positioned over a vacuum flask. The filter is rinsed four times with buffer. The filter is then counted in a NaI (T1) scintillation counter. Specific binding is experimentally determined from the difference between counts bound in the absence and presence of 1 µM atropine.

This same procedure can be used for any of the radio-labeled derivatives of the invention.

**Claims**

1. A compound of the formula:

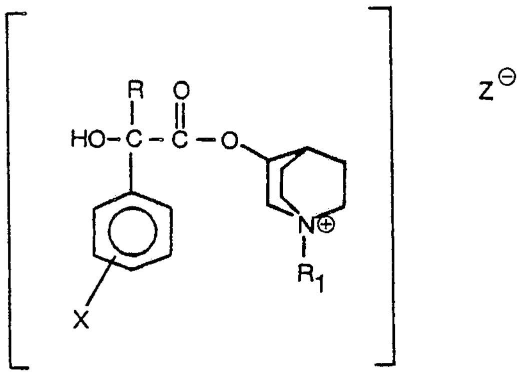

Wherein
R is phenyl, cyclopentyl or cyclohexyl,
$R_1$ is H or $CH_3$,
X is in the ortho-, meta- or para- position, and is selected from the $^{125}I$, $^{123}I$, I, $^{18}F$, $^{77}Br$, $^{75}Br$, $NH_2$, and

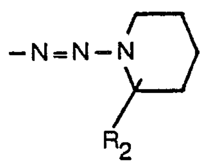

wherein $R_2$ is in the 2, 3, or 4, position and is selected from H and $CH_3$, and,
$Z^{\ominus}$ is an anion; or the free amine form thereof.

2. A compound of the formula:

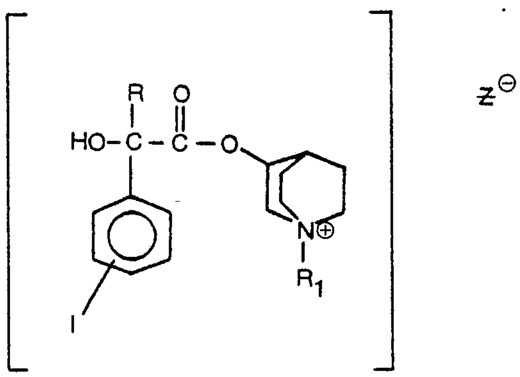

Wherein
R is phenyl, cyclopentyl or cyclohexyl,
$R_1$ is H or $CH_3$,
X is in the ortho-, meta- or para- position, and
$Z^{\ominus}$ is an anion; or the free amine form thereof.

3. The compound of Claim 2, wherein said I is the meta-position and $R_1$ is H.
4. The compound of Claim 2 wherein said I is in the meta-position and $R_1$ is $CH_3$.
5. The compound of Claim 2 wherein said I is in the para-position and $R_1$ is CH.
6. The compound of Claim 2 wherein said I is in the para-position and $R_1$ is $CH_3$.
7. The compound of Claim 2 wherein I is in the ortho-position and $R_1$ is H.
8. The compound of Claim 2 wherein I is in the ortho-position and $R_1$ is $CH_3$.
9. A compound of the formula:

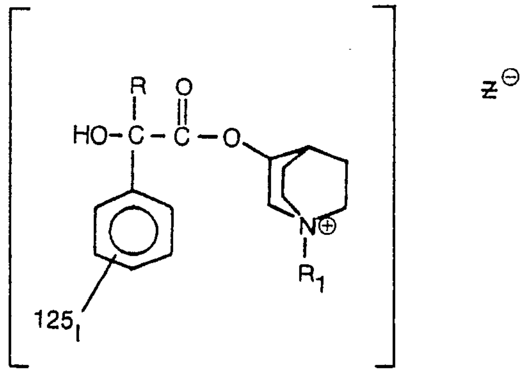

Wherein

R is phenyl, cyclopentyl or cyclohexyl,

$R_1$ is H or $CH_3$,

$^{125}I$, is in the ortho-, meta- or para- position, and

$Z^{\ominus}$ is an anion; or the free amine form thereof.

10. The compound of Claim 9 wherein said $^{125}I$ is in the meta-position and $R_1$ is H.

11. The compound of Claim 9 wherein $^{125}I$ is in the meta-position and $R_1$ is $CH_3$.

12. The compound of Claim 9 wherein said $^{125}I$ is in the para-position and $R_1$ is H.

13. The compound of Claim 9 wherein said $^{125}I$ is in the para-position and $R_1$ is $CH_3$.

14. The compound of Claim 9 wherein $^{125}I$ is in the ortho-position and $R_1$ is H.

15. The compound of Claim 9 wherein $^{125}I$ is in the ortho-position and $R_1$ is $CH_3$.

16. A compound of the formula:

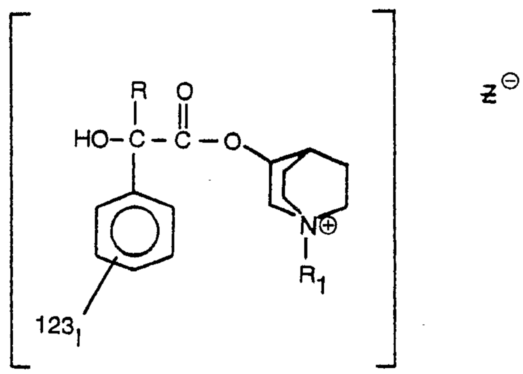

Wherein

R is phenyl, cyclopentyl or cyclohexyl,

$R_1$ is H or $CH_3$,

$^{123}I$ is in the ortho-, meta- or para- position, and

$Z^{\ominus}$ is an anion; or the free amine form thereof.

17. The compound of Claim 16 wherein said $^{123}I$ is in the meta-position and $R_1$ is H.

18. The compound of Claim 16 wherein said $^{123}I$ is in the meta-position and $R_1$ is $CH_3$.

19. The compound of Claim 16 wherein said $^{123}I$ is in the para-position and $R_1$ is H.

20. The compound of Claim 16 wherein said $^{123}I$ is in the para-position and $R_1$ is $CH_3$.

21. The compound of Claim 16 wherein said $^{123}I$ is in the ortho-position and $R_1$ is H.

22. The compound of Claim 16 wherein said $^{123}I$ is in the ortho-position and $R_1$ is $CH_3$.

23. A compound of the formula:

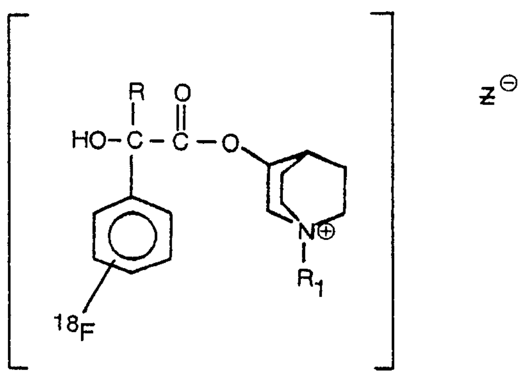

Wherein

R is phenyl, cyclopentyl or cyclohexyl,

$R_1$ is H or $CH_3$,

$^{18}F$ is in the ortho-, meta- or para- position, and

$Z^{\ominus}$ is anion; or the free amine form thereof.

24. The compound of Claim 23 wherein said $^{18}F$ is in the meta-position and $R_1$ is H.
25. The compound of Claim 23 wherein said $^{18}F$ is in the meta-position and $R_1$ is $CH_3$.
26. The compound of Claim 23 wherein said $^{18}F$ is in the para-position and $R_1$ is H.
27. The compound of Claim 23 wherein said $^{18}F$ is in the para-position and $R_1$ is $CH_3$.
28. The compound of Claim 23 wherein said $^{18}F$ is in the ortho-position and $R_1$ is H.
29. The compound of Claim 23 wherein said $^{18}F$ is in the ortho-position and $R_1$ is $CH_3$.
30. A compound of the formula:

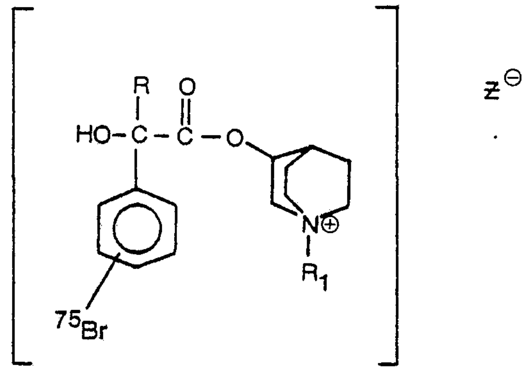

Wherein R is phenyl, cyclopentyl or cyclohexyl,

$R_1$ is H or $Ch_3$,

$^{75}Br$ is in the ortho-, meta- or para-position, and

$Z^{\ominus}$ is an anion; or the free amine form thereof.

31. The compound of Claim 30 wherein said $^{75}Br$ is in the meta-position and $R_1$ is H.
32. The compound of Claim 30 wherein said $^{75}Br$ is in the meta-position and $R_1$ is $CH_3$.
33. The compound of Claim 30 wherein said $^{75}Br$ is in the para-position and $R_1$ is H.
34. The compound of Claim 30 wherein said $^{75}Br$ is in the para-position and $R_1$ is $CH_3$.
35. The compound of Claim 30 wherein said $^{75}Br$ is in the ortho-position and $R_1$ is H.
36. The compound of Claim 30 wherein said $^{75}Br$ is in the ortho-position and $R_1$ is $CH_3$.
37. A compound of the formula:

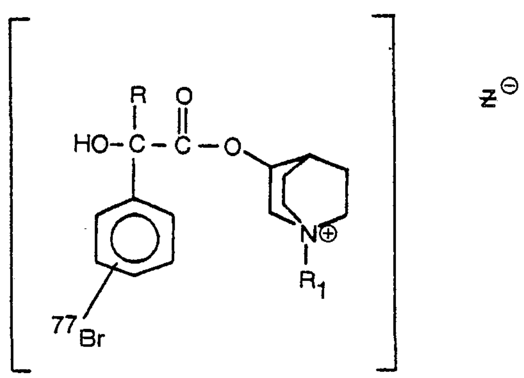

Wherein R is phenyl, cyclopentyl or cyclohexyl,

$R_1$ is H or $CH_3$,

$^{77}Br$ is in the ortho-, meta- or para-position, and

$Z^{\ominus}$ is an anion; or the free amine form thereof.

38. The compound of Claim 37 wherein said $^{77}Br$ is in the meta-position and $R_1$ is H.
39. The compound of Claim 37 wherein said $^{77}Br$ is in the meta-position and $R_1$ is $CH_3$.
40. The compound of Claim 37 wherein said $^{77}Br$ is in the para-position and $R_1$ is H.
41. The compound of Claim 37 wherein said $^{77}Br$ is in the para-position and $R_1$ is $CH_3$.
42. The compound of Claim 37 wherein said $^{77}Br$ is in the ortho-position and $R_1$ is H.
43. The compound of Claim 37 wherein said $^{77}Br$ is in the ortho-position and $R_1$ is $CH_3$.
44. A compound of the formula:

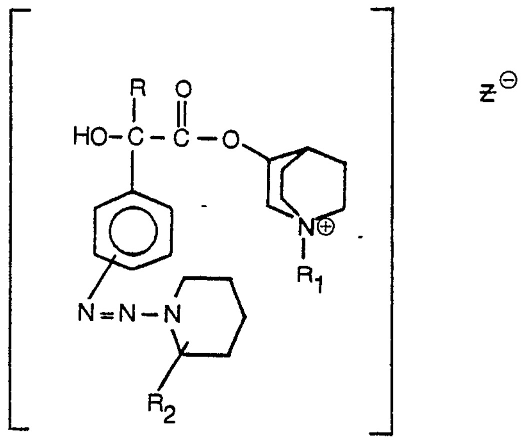

Wherein R is phenyl, cyclopentyl or cyclohexyl,
$R_1$ is H or $CH_3$,
the triazene group is in the ortho-, meta- or para-position, $R_2$ is H or $CH_3$ in the 2, 3, or 4 position, and $Z^{\ominus}$ is an anion; or the free amine form thereof.

45. The compound of Claim 44 wherein said triazene group is in the meta-position and $R_1$ is H.
46. The compound of Claim 44 wherein said triazine group is in the meta-position and $R_1$ is $CH_3$.
47. The compound of Claim 44 wherein said triazene group is in the para-position and $R_1$ is H.
48. The compound of Claim 44 wherein said triazine group is in the para-position and $R_1$ is $CH_3$.
49. The compound of Claim 44 wherein said triazene group is in the ortho-position and $R_1$ is H.
50. The compound of Claim 44 wherein said triazine group is in the ortho-position and $R_1$ is $CH_3$.
51. A compound of the formula:

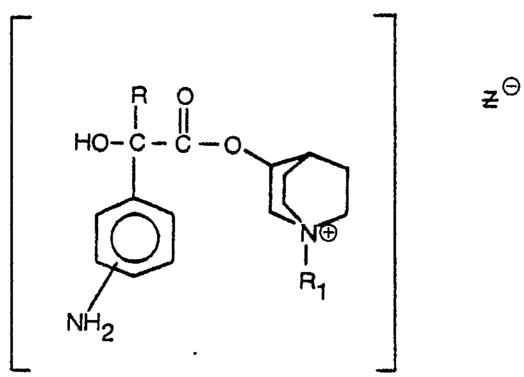

Wherein R is phenyl, cyclopentyl or cyclohexyl,
$R_1$ is H or $CH_3$,
$NH_2$ is in the ortho-, meta- or para-position, and
$Z^{\ominus}$ is an anion; or the free amine form thereof.

52. The compound of Claim 51 wherein said $NH_2$ is in the meta-position and $R_1$ is H.
53. The compound of Claim 51 wherein said $NH_2$ is in the meta-position and $R_1$ is $CH_3$.
54. The compound of Claim 51 wherein said $NH_2$ is in the para-position and $R_1$ is H.
55. The compound of Claim 51 wherein said $NH_2$ is in the para-position and $R_1$ is $CH_3$.
56. The compound of Claim 51 wherein said $NH_2$ is in the ortho-position and $R_1$ is H.
57. The compound of Claim 51 wherein said $NH_2$ is in the ortho-position and $R_1$ is $CH_3$.
58. A method for preparing a compound of Claim 51 comprising reacting a derivative of an aminobenzilic acid with a quinuclidin-3-ol to produce a quinuclidinyl aminobenzilate.
59. A method for preparing a compound of Claim 44 comprising reacting a derivative of an aminobenzilic acid with a quinuclidin-3-ol to produce a quinuclidinyl aminobenzilate and converting the free amine group to a triazene group.
60. A method for preparing a compound of Claim 1 comprising reacting a derivative of an aminobenzilic acid with a quinuclidin-3-ol to produce a quinuclidinyl aminobenzilate, converting the free amino group to a triazene group, reacting the triazene derivative with a halogenide or radiohalogenide to form 3-quinuclidinyl halo benzilate.
61. A composition suitable for the assay of muscarinic cholinergic receptors in tissue comprising a gamma-emitting radioisotope containing compound of Claim 1 and a pharmaceutically acceptable carrier therefor.

62. A composition of Claim 61 wherein said gamma-emitter is $^{125}I$.
63. The composition of Claim 61 wherein said gamma-emitter is $^{123}I$.
64. The composition of Claim 61 wherein said gamma-emitter is $^{18}F$.
65. The composition of Claim 61 wherein said gamma-emitter is $^{75}Br$.
66. The composition of Claim 61 wherein said gamma-emitter is $^{77}Br$.
67. A composition in unit dosage form suitable for use in a method for the external imaging or radio-assay of tissue containing muscarinic cholinergic receptors comprising a gamma-emitting radioisotope containing compound of Claim 1 and a pharmaceutically acceptable carrier therefor.

**Patentansprüche**

1. Verbindung der Formel

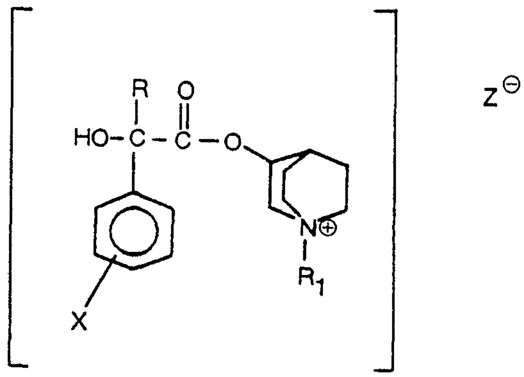

in der
R Phenyl, Cyclopentyl oder Cyclohexyl ist,
$R_1$ H oder $CH_3$ ist,
X in der ortho-, meta- oder para-Stellung steht und gewählt ist aus $^{125}J$, $^{123}J$, J, $^{18}F$, $^{77}Br$, $^{75}Br$, $NH_2$ und

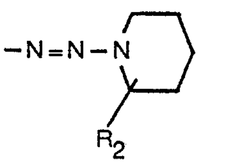

in der $R_2$ die 2-, 3- oder 4-Stellung ist und gewählt ist aus H und $CH_3$, und
$Z^{\ominus}$ ein Anion ist, oder die freie Amin-Form davon ist.
2. Verbindung der Formel

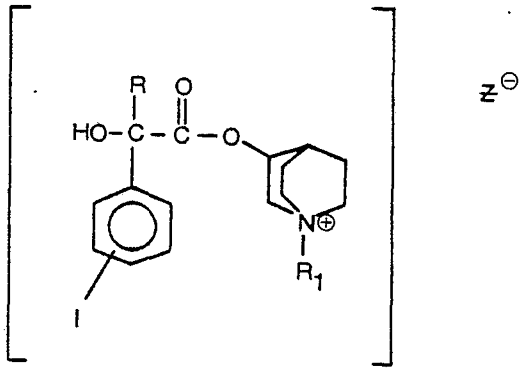

in der
R Phenyl, Cyclopentyl oder Cyclohexyl ist,
$R_1$ H oder $CH_3$ ist,
J in der ortho-, meta- oder para-Stellung steht und
$Z^{\ominus}$ ein Anion ist, oder die freie Amin-Form davon.
3. Verbindung nach Anspruch 2, in der J in der meta-Stellung steht und $R_1$ H ist.
4. Verbindung nach Anspruch 2, in der J in der meta-Stellung steht und $R_1$ $CH_3$ ist.

5. Verbindung nach Anspruch 2, in der J in der para-Stellung steht und $R_1$ H ist.
6. Verbindung nach Anspruch 2, in der J in der para-Stellung steht und $R_1$ $CH_3$ ist.
7. Verbindung nach Anspruch 2, in der J in der ortho-Stellung steht und $R_1$ H ist.
8. Verbindung nach Anspruch 2, in der J in der ortho-Stellung steht und $R_1$ $CH_3$ ist.
9. Verbindung der Formel

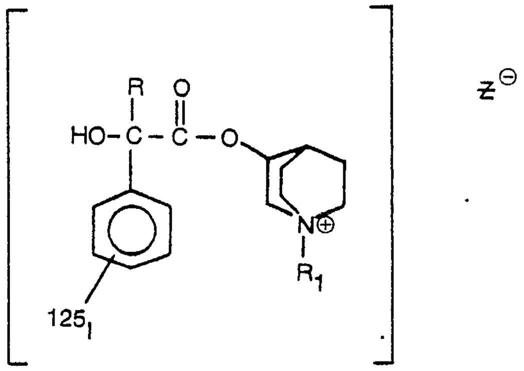

in der
R Phenyl, Cyclopentyl oder Cyclohexyl ist,
$R_1$ H oder $CH_3$ ist,
$^{125}I$ in der ortho-, meta- oder para-Stellung steht, und
$Z^{\ominus}$ ein Anion ist, oder die freie Amin-Form davon.

10. Verbindung nach Anspruch 9, in der das $^{125}J$ in der meta-Stellung steht und $R_1$ H ist.
11. Verbindung nach Anspruch 9, in der das $^{125}J$ in der meta-Stellung steht und $R_1$ $CH_3$ ist.
12. Verbindung nach Anspruch 9, in der das $^{125}J$ in der para-Stellung steht und $R_1$ H ist.
13. Verbindung nach Anspruch 9, in der das $^{125}J$ in der para-Stellung steht und $R_1$ $CH_3$ ist.
14. Verbindung nach Anspruch 9, in der das $^{125}J$ in der ortho-Stellung steht und $R_1$ H ist.
15. Verbindung nach Anspruch 9, in der das $^{125}J$ in der ortho-Stellung steht und $R_1$ $CH_3$ ist.
16. Verbindung der Formel

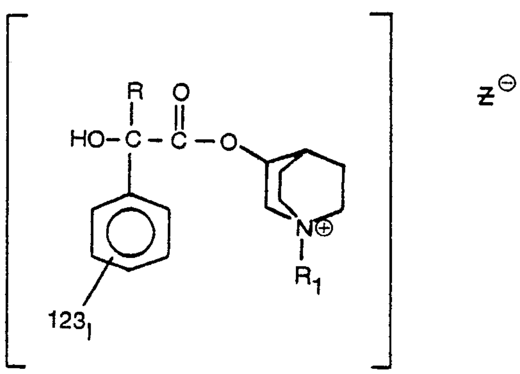

in der
R Phenyl, Cyclopentyl oder Cyclohexyl ist,
$R_1$ H oder $CH_3$ ist,
$^{123}I$ in der ortho-, meta- oder para-Stellung steht, und
$Z^{\ominus}$ ein Anion ist, oder freie Amin-Form davon.

17. Verbindung nach Anspruch 16, in der das $^{123}J$ in der meta-Stellung steht und $R_1$ H ist.
18. Verbindung nach Anspruch 16, in der das $^{123}J$ in der meta-Stellung steht und $R_1$ $CH_3$ ist.
19. Verbindung nach Anspruch 16, in der das $^{123}J$ in der para-Stellung steht und $R_1$ H ist.
20. Verbindung nach Anspruch 16, in der das $^{123}J$ in der para-Stellung steht und $R_1$ $CH_3$ ist.
21. Verbindung nach Anspruch 16, in der das $^{123}J$ in der ortho-Stellung steht und $R_1$ H ist.
22. Verbindung nach Anspruch 16, in der das $^{123}J$ in der ortho-Stellung steht und $R_1$ $CH_3$ ist.
23. Verbindung der Formel

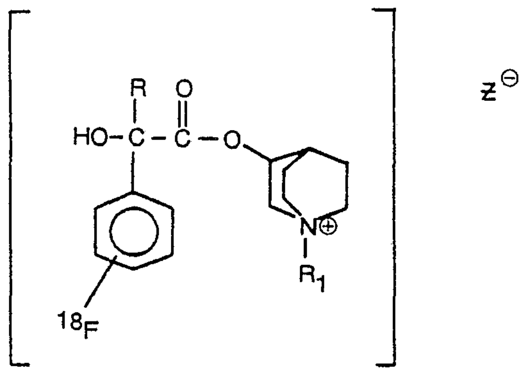

in der
R Phenyl, Cyclopentyl oder Cyclohexyl ist,
$R_1$ H oder $CH_3$ ist,
$^{18}$I in der ortho-, meta- oder para-Stellung steht, und
$Z^{\ominus}$ ein Anion ist, oder die freie Amin-Form davon.

24. Verbindung nach Anspruch 23, in der das $^{18}$F in der meta-Stellung steht und $R_1$ H ist.
25. Verbindung nach Anspruch 23, in der das $^{18}$F in der meta-Stellung steht und $R_1$ $CH_3$ ist.
26. Verbindung nach Anspruch 23, in der das $^{18}$F in der para-Stellung steht und $R_1$ H ist.
27. Verbindung nach Anspruch 23, in der das $^{18}$F in der para-Stellung steht und $R_1$ $CH_3$ ist.
28. Verbindung nach Anspruch 23, in der das $^{18}$F in der ortho-Stellung steht und $R_1$ H ist.
29. Verbindung nach Anspruch 23, in der das $^{18}$F in der ortho-Stellung steht und $R_1$ $CH_3$ ist.
30. Verbindung der Formel

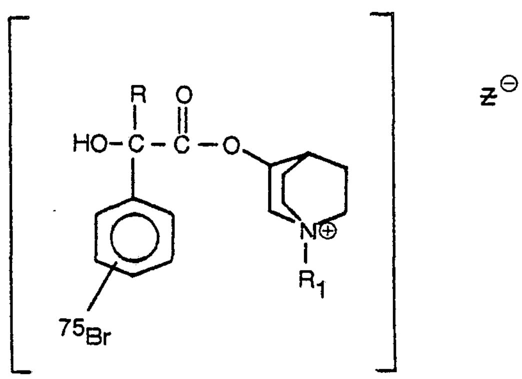

in der
R Phenyl, Cyclopentyl oder Cyclohexyl ist,
$R_1$ H oder $CH_3$ ist,
$^{75}$Br in der ortho-, meta- oder para-Stellung steht, und
$Z^{\ominus}$ ein Anion ist, oder die freie Amin-Form davon.

31. Verbindung nach Anspruch 30, in der das $^{75}$Br in der meta-Stellung steht und $R_1$ H ist.
32. Verbindung nach Anspruch 30, in der das $^{75}$Br in der meta-Stellung steht und $R_1$ $CH_3$ ist.
33. Verbindung nach Anspruch 30, in der das $^{75}$Br in der para-Stellung steht und $R_1$ H ist.
34. Verbindung nach Anspruch 30, in der das $^{75}$Br in der para-Stellung steht und $R_1$ $CH_3$ ist.
35. Verbindung nach Anspruch 30, in der das $^{75}$Br in der ortho-Stellung steht und $R_1$ H ist.
36. Verbindung nach Anspruch 30, in der das $^{75}$Br in der ortho-Stellung steht und $R_1$ $CH_3$ ist.
37. Verbindung der Formel

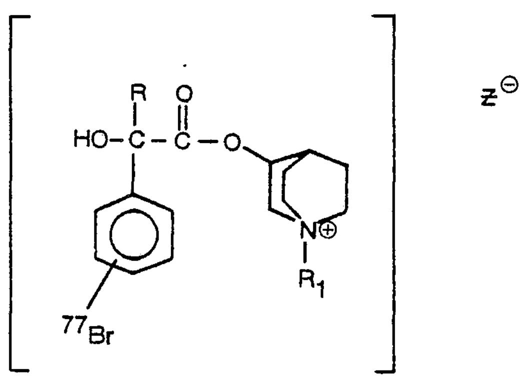

in der

R Phenyl, Cyclopentyl oder Cyclohexyl ist,

$R_1$ H oder $CH_3$ ist,

$^{77}$Br in der ortho-, meta- oder para-Stellung steht, und

$Z^{\ominus}$ ein Anion ist, oder freie Amin-Form davon.

38. Verbindung nach Anspruch 37, in der das $^{77}$Br in der meta-Stellung steht und $R_1$ H ist.
39. Verbindung nach Anspruch 37, in der das $^{77}$Br in der meta-Stellung steht und $R_1$ $CH_3$ ist.
40. Verbindung nach Anspruch 37, in der das $^{77}$Br in der para-Stellung steht und $R_1$ H ist.
41. Verbindung nach Anspruch 37, in der das $^{77}$Br in der para-Stellung steht und $R_1$ $CH_3$ ist.
42. Verbindung nach Anspruch 37, in der das $^{77}$Br in der ortho-Stellung steht und $R_1$ H ist.
43. Verbindung nach Anspruch 37, in der das $^{77}$Br in der ortho-Stellung steht und $R_1$ $CH_3$ ist.
44. Verbindung der Formel

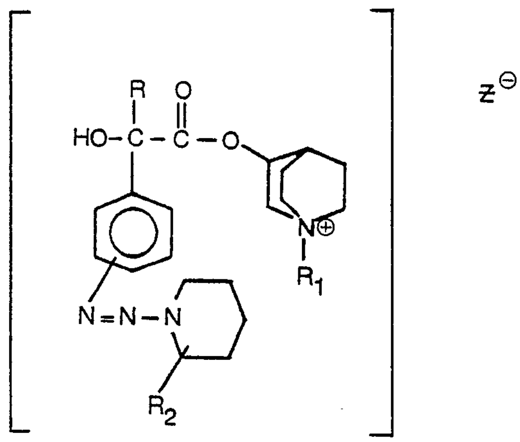

in der

R Phenyl, Cyclopentyl oder Cyclohexyl ist,

$R_1$ H oder $CH_3$ ist,

die Triazen-Gruppe in der ortho-, meta- oder para-Stellung steht,

$R_2$ H oder $CH_3$ ist und in der 2-, 3- oder 4-Stellung steht, und

$Z^{\ominus}$ ein Anion ist, oder die freie Amin-Form davon.

45. Verbindung nach Anspruch 44, in der die Triazen-Gruppe in der meta-Stellung und $R_1$ H ist.
46. Verbindung nach Anspruch 44, in der die Triazen-Gruppe in der meta-Stellung und $R_1$ $CH_3$ ist.
47. Verbindung nach Anspruch 44, in der die Triazen-Gruppe in der para-Stellung und $R_1$ H ist.
48. Verbindung nach Anspruch 44, in der die Triazen-Gruppe in der para-Stellung und $R_1$ $CH_3$ ist.
49. Verbindung nach Anspruch 44, in der die Triazen-Gruppe in der ortho-Stellung und $R_1$ H ist.
50. Verbindung nach Anspruch 44, in der die Triazen-Gruppe in der ortho-Stellung und $R_1$ $CH_3$ ist.
51. Verbindung der Formel

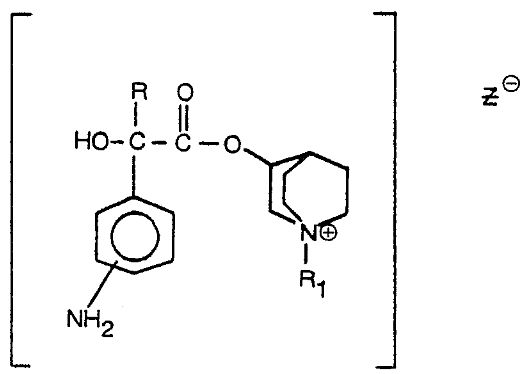

in der

R Phenyl, Cyclopentyl oder Cyclohexyl ist,

$R_1$ H oder $CH_3$ ist,

$NH_2$ in der ortho-, meta- oder para-Stellung steht, und

$Z^{\ominus}$ ein Anion ist, oder die freie Amin-Form ist.

52. Verbindung nach Anspruch 51, in der das $NH_2$ in der meta-Stellung steht und $R_1$ H ist.
53. Verbindung nach Anspruch 51, in der das $NH_2$ in der meta-Stellung steht und $R_1$ $CH_3$ ist.
54. Verbindung nach Anspruch 51, in der das $NH_2$ in der para-Stellung steht und $R_1$ H ist.

55. Verbindung nach Anspruch 51, in der das $NH_2$ in der para-Stellung steht und $R_1$ $CH_3$ ist.

56. Verbindung nach Anspruch 51, in der das $NH_2$ in der ortho-Stellung steht und $R_1$ H ist.

57. Verbindung nach Anspruch 51, in der das $NH_2$ in der ortho-Stellung steht und $R_1$ $CH_3$ ist.

58. Verfahren zur Herstellung einer Verbindung nach Anspruch 51, dadurch gekennzeichnet, daß man ein Derivat einer Aminobenzilsäure mit einem Chinuclidin-3-ol zu einem Chinuclidinyl-aminobenzilat umsetzt.

59. Verfahren zur Herstellung einer Verbindung nach Anspruch 44, dadurch gekennzeichnet, daß man ein Derivat einer Aminobenzilsäure mit einem Chinuclidin-3-ol zu einem Chinuclidinyl-aminobenzilat umsetzt und die freie Aminogruppe in eine Triazengruppe umwandelt.

60. Verfahren zur Herstellung einer Verbindung nach Anspruch 1, dadurch gekennzeichnet, daß man ein Derivat einer Aminobenzilsäure mit einem Chinuclidin-3-ol zu einem Chinuclidinyl-aminobenzilat umsetzt, die freie Aminogruppe in eine Triazengruppe mit einem Halogenid oder Radiohalogenid zu 3-Chinuclidinyl-halogen-benzilat umsetzt.

61. Mittel zur Ermittlung muscarin-cholinergischer Receptoren im Gewebe, dadurch gekennzeichnet, daß es eine ein gamma-emittierendes Radiosotop enthaltende Verbindung nach Anspruch 1 und einem pharmazeutisch akzeptablen Träger dafür enthält.

62. Mittel nach Anspruch 61, dadurch gekennzeichnet, daß der gamma-Emitter $^{125}J$ ist.

63. Mittel nach Anspruch 61, dadurch gekennzeichnet, daß der gamma-Emitter $^{123}J$ ist.

64. Mittel nach Anspruch 61, dadurch gekennzeichnet, daß der gamma-Emitter $^{18}F$ ist.

65. Mittel nach Anspruch 61, dadurch gekennzeichnet, daß der gamma-Emitter $^{75}Br$ ist.

66. Mittel nach Anspruch 61, dadurch gekennzeichnet, daß der gamma-Emitter $^{77}Br$ ist.

67. Mittel in Einheitsdosierungsform zur Verwendung in einem Verfahren zur externen Verbildlichung oder Radio-Prüfung von Gewebe, dadurch gekennzeichnet, daß es eine ein gamma-emittierendes Radioisotop enthaltende Verbindung gemaß Anspruch 1 und einen pharmazeutisch akzeptablen Träger dafür enthält.

**Revendications**

1. Composé de formule:

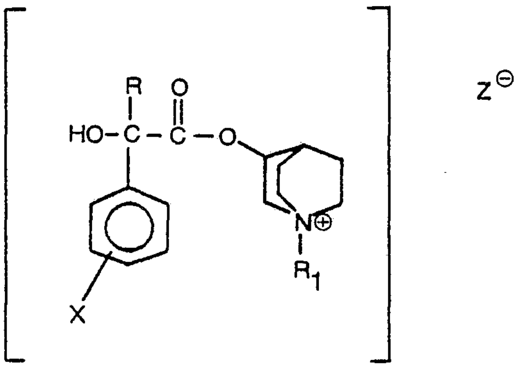

dans laquelle

R est un groupe phényle, cyclopentyle ou cyclohexyl,

$R_1$ représente H ou le groupe $CH_3$,

X est en position ortho, méta ou para et est choisi entre $^{125}I$, $^{123}I$, I, $^{18}F$, $^{77}Br$, $^{75}Br$, $NH_2$ et un groupe de formule

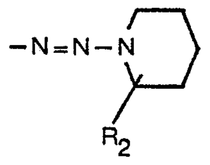

dans laquelle $R_2$ est en position 2, 3 ou 4 et est choisi entre H et $CH_3$ et

$Z^{\ominus}$ est un anion; ou la forme amine libre de ce composé.

2. Composé de formule:

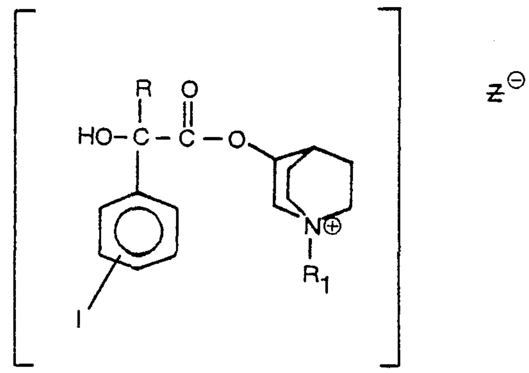

dans laquelle

R est un groupe phényle, cyclopentyle ou cyclohexyle,

$R_1$ représente H ou $CH_3$,

I est en position ortho, méta ou para, et

$Z^\ominus$ est un anion; ou la forme amine libre de ce composé.

3. Composé suivant la revendication 2, dans lequel I est en position méta et $R_1$ représente H.
4. Composé suivant la revendication 2, dans lequel I est en position méta et $R_1$ est un groupe $CH_3$.
5. Composé suivant la revendication 2, dans lequel I est en position para et $R_1$ représente H.
6. Composé suivant la revendication 2, dans lequel I est en position para et $R_1$ est un groupe $CH_3$.
7. Composé suivant la revendication 2, dans lequel I est en position ortho et $R_1$ représente H.
8. Composé suivant la revendication 2, dans lequel I est en position ortho et $R_1$ est un groupe $CH_3$.
9. Composé de formule:

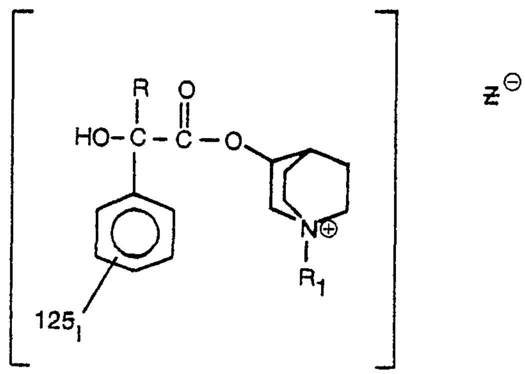

dans laquelle

R est un groupe phényle, cyclopentyle ou cyclohexyle,

$R_1$ représente H ou $CH_3$,

$^{125}I$ est en position ortho, méta ou para, et

$Z^\ominus$ est un anion; ou la forme amine libre de ce composé.

10. Composé suivant la revendication 9, dans lequel $^{125}I$ est en position méta et $R_1$ représente H.
11. Composé suivant la revendication 9, dans lequel $^{125}I$ est en position méta et $R_1$ est un groupe $CH_3$.
12. Composé suivant la revendication 9, dans lequel $^{125}I$ est en position para et $R_1$ représente H.
13. Composé suivant la revendication 9, dans lequel $^{125}I$ est en position para et $R_1$ est un groupe $CH_3$.
14. Composé suivant la revendication 9, dans lequel $^{125}I$ est en position ortho et $R_1$ représente H.
15. Composé suivant la revendication 9, dans lequel $^{125}I$ est en position ortho et $R_1$ est un groupe $CH_3$.
16. Composé de formule:

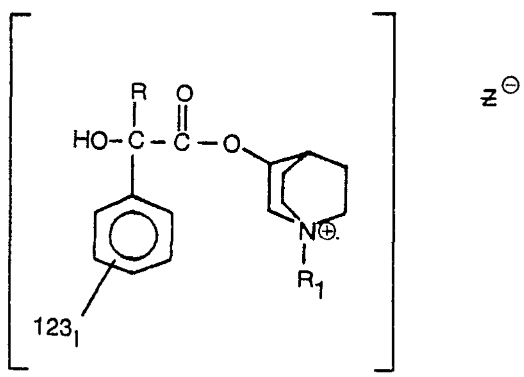

dans laquelle

R est un groupe phényle, cyclopentyle ou cyclohexyle,

$R_1$ rperésente H ou $CH_3$,

$^{123}I$ est en position ortho, méta ou para, et

$Z^{\ominus}$ est un anion; ou la forme amine libre de ce composé.

17. Composé suivant la revendication 16, dans lequel $^{123}I$ est en position méta et $R_1$ représente H.
18. Composé suivant la revendication 16, dans lequel $^{123}I$ est en position méta et $R_1$ représente $CH_3$.
19. Composé suivant la revendication 16, dans lequel $^{123}I$ est en position para et $R_1$ représente H.
20. Composé suivant la revendication 16, dans lequel $^{123}I$ est en position para et $R_1$ représente $CH_3$.
21. Composé suivant la revendication 16, dans lequel $^{123}I$ est en position ortho et $R_1$ représente H.
22. Composé suivant la revendication 16, dans lequel $^{123}I$ est en position ortho et $R_1$ représente $CH_3$.
23. Composé de formule:

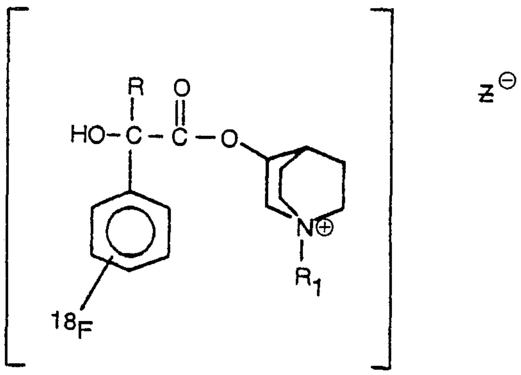

dans laquelle

R est un groupe phényle, cyclopentyle ou cyclohexyle,

$R_1$ représente H ou $CH_3$,

$^{18}F$ est en position ortho, méta ou para, et

$Z^{\ominus}$ est un anion; ou la forme amine libre de ce composé.

24. Composé suivant la revendication 23, dans lequel $^{18}F$ est en position méta et $R_1$ représente H.
25. Composé suivant la revendication 23, dans lequel $^{18}F$ est en position méta et $R_1$ représente $CH_3$.
26. Composé suivant la revendication 23, dans lequel $^{18}F$ est en position para et $R_1$ représente H.
27. Composé suivant la revendication 23, dans lequel $^{18}F$ est en position para et $R_1$ représente $CH_3$.
28. Composé suivant la revendication 23, dans lequel $^{18}F$ est en position ortho et $R_1$ représente H.
29. Composé suivant la revendication 23, dans lequel $^{18}F$ est en position ortho et $R_1$ représente $CH_3$.
30. Composé de formule:

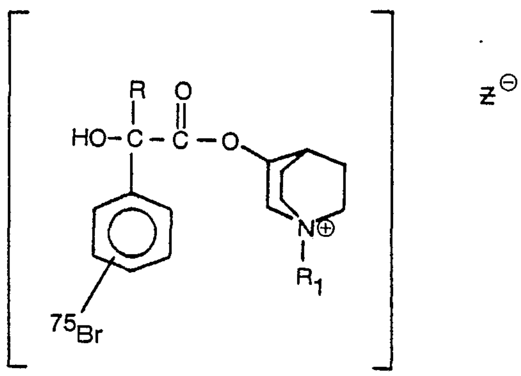

dans laquelle

R est un groupe phényle, cyclopentyle ou cyclohexyle,

$R_1$ représente H ou $CH_3$,

$^{75}Br$ est en position ortho, méta ou para, et

$Z^{\ominus}$ est un amine; ou la forme amine libre de ce composé.

31. Composé suivant la revendication 30, dans lequel $^{75}Br$ est en position méta et $R_1$ représente H.
32. Composé suivant la revendication 30, dans lequel $^{75}Br$ est en position méta et $R_1$ représente $CH_3$.
33. Composé suivant la revendication 30, dans lequel $^{75}Br$ est en position para et $R_1$ représente H.
34. Composé suivant la revendication 30, dans lequel $^{75}Br$ est en position para et $R_1$ représente $CH_3$.
35. Composé suivant la revendication 30, dans lequel $^{75}Br$ est en position ortho et $R_1$ représente H.

36. Composé suivant la revendication 30, dans lequel $^{75}Br$ est en position ortho et $R_1$ représente $CH_3$.

37. Composé de formule:

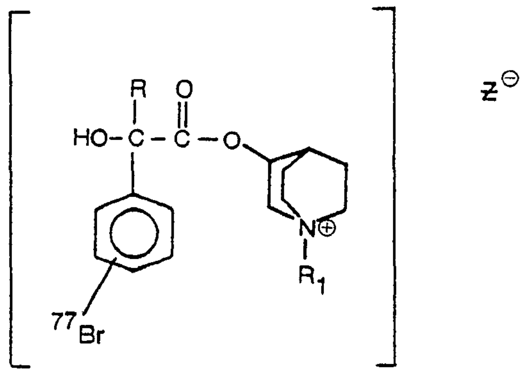

dans laquelle

R est un groupe phényle, cyclopentyle ou cyclohexyle,

$R_1$ représente H ou $CH_3$,

$^{77}Br$ est en position ortho, méta ou para, et

$Z^{\ominus}$ est un anion; ou la forme amine libre de ce composé.

38. Composé suivant la revendication 37, dans lequel $^{77}Br$ est en position méta et $R_1$ représente H.

39. Composé suivant la revendication 37, dans lequel $^{77}Br$ est en position méta et $R_1$ représente $CH_3$.

40. Composé suivant la revendication 37, dans lequel $^{77}Br$ est en position para et $R_1$ représente H.

41. Composé suivant la revendication 37, dans lequel $^{77}Br$ est en position para et $R_1$ représente $CH_3$.

42. Composé suivant la revendication 37, dans lequel $^{77}Br$ est en position ortho et $R_1$ représente H.

43. Composé suivant la revendication 37, dans lequel $^{77}Br$ est en position ortho et $R_1$ représente $CH_3$.

44. Composé de formule:

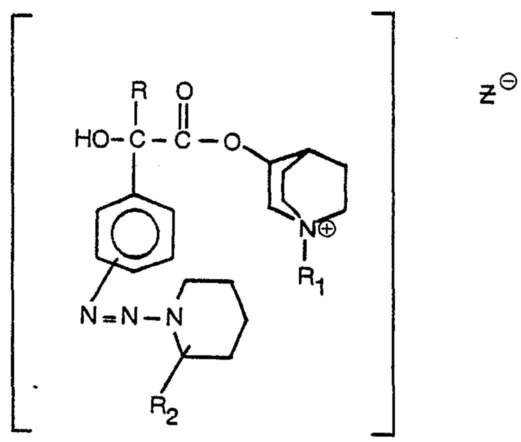

dans laquelle

R est un groupe phényle, cyclopentyle ou cyclohexyle,

$R_1$ représente H ou $CH_3$,

le groupe triazène est en position ortho, méta ou para,

$R_2$représente H ou $CH_3$ en position 2, 3 ou 4, et

$Z^{\ominus}$ est un anion; ou la forme amine libre de ce composé.

45. Composé suivant la revendication 44, dans lequel le groupe triazène est en position méta et $R_1$ représente H.

46. Composé suivant la revendication 44, dans lequel le groupe triazène est en position méta et $R_1$ représente $CH_3$.

47. Composé suivant la revendication 44, dans lequel le groupe triazène est en position para et $R_1$ représente H.

48. Composé suivant la revendication 44, dans lequel le groupe triazène est en position para et $R_1$ représente $CH_3$.

49. Composé suivant la revendication 44, dans lequel le groupe triazène est en position ortho et $R_1$ représente H.

50. Composé suivant la revendication 44, dans lequel le groupe triazène est en position ortho et $R_1$ représente $CH_3$.

51. Composé de formule:

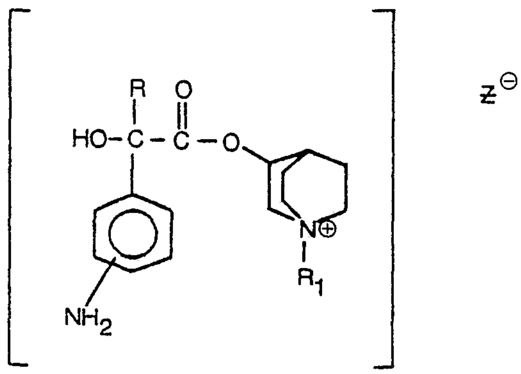

dans laquelle

R est un groupe phényle, cyclopentyle ou cyclohexyle,

$R_1$ représente H ou $CH_3$,

$NH_2$ est en position ortho, méta ou para, et

$Z^{\ominus}$ est un anion; ou la forme amine libre de ce composé.

52. Composé suivant la revendication 51, dans lequel le groupe $NH_2$ est en position méta et $R_1$ représente H.

53. Composé suivant la revendication 51, dans lequel le groupe $NH_2$ est en position méta et $R_1$ représente $CH_3$.

54. Composé suivant la revendication 51, dans lequel $NH_2$ est en position para et $R_1$ représente H.

55. Composé suivant la revendication 51, dans lequel $NH_2$ est en position para et $R_1$ représente $CH_3$.

56. Composé suivant la revendication 51, dans lequel $NH_2$ est en position ortho et $R_1$ représente H.

57. Composé suivant la revendication 51, dans lequel $NH_2$ est en position ortho et $R_1$ représente $CH_3$.

58. Procédé de préparation d'un composé suivant la revendication 51, qui consiste à faire réagir un dérivé d'un acide aminobenzilique avec un quinuclidine-3-ol pour produire un aminobenzilate de quinuclidinyle.

59. Procédé de préparation d'un composé suivant la revendication 44, qui consiste à faire réagir un dérivé d'un acide aminobenzilique avec un quinuclidine-3-ol pour produire un aminobenzilate de quinuclidinyle et à transformer le groupe amino libre en un groupe triazène.

60. Procédé de préparation d'un composé suivant la revendication 1, qui consiste à faire réagir un dérivé d'un acide aminobenzilique avec un quinuclidine-3-ol pour produire un aminobenzilate de quinuclidinyle, à convertir le groupe amino libre en un groupe triazène, à faire réagir le dérivé triazénique avec un halogénure ou un radiohalogénure pour former l'halogénobenzilate de 3-quinuclidinyle.

61. Composition pouvant être utilisée pour l'analyse de récepteurs cholinergiques muscariniques dans un tissu, comprenant un composé contenant un radioisotope gammagène suivant la revendication 1 et un support pharmaceutiquement acceptable approprié.

62. Composition suivant la revendication 61, dans laquelle l'émetteur de rayons gamma est $^{125}I$.

63. Composition suivant la revendication 61, dans laquelle l'émetteur de rayons gamma est $^{123}I$.

64. Composition suivant la revendication 61, dans laquelle l'émetteur de rayons gamma est $^{18}F$.

65. Composition suivant la revendication 61, dans laquelle l'émetteur de rayons gamma est $^{75}Br$.

66. Composition suivant la revendication 61, dans laquelle l'émetteur de rayons gamma est $^{77}Br$.

67. Composition sous la forme dosée unitaire destinée à être utilisée dans un procédé de visualisation externe ou un radio-essai de tissu contenant des récepteurs cholinergiques muscariniques, comprenant un composé contenant un radio-isotope gammagène suivant la revendication 1 et un support pharmaceutiquement acceptable approprié.